# NEW EUROPEAN PATENT SPECIFICATION

(11) **EP 1 366 755 B2**
(45) Date of publication and mention of the opposition decision: **04.04.2007**
(45) Mention of the grant of the patent: 15.09.2004
(21) Application number: 03011496.1
(22) Date of filing: 21.05.2003
(51) Int. Cl.: A61K 8/34, A61K 8/41, A61Q 5/12, A61K 8/36, A61Q 5/10

(54) **Treatment composition for reducing bleeding of acidic dyes from colored human hair**
Behandlungsmittel von gefärbten menschlichen Haaren zur Ausblutungsverminderung von Säurefarbstoffen
Composition de traitement des cheveux humains colorés pour diminuer la migration de colorants acides

(30) Priority: 28.05.2002 DE 10223559; 28.05.2002 DE 20208254 U
(43) Date of publication of application: 03.12.2003
(73) Proprietor: KPSS-Kao Professional Salon Services GmbH, 64297 Darmstadt (DE)
(72) Inventor: Nöcker, Bernd, Dr., 64372 Ober-Ramstadt (DE); Vega-Carrascal, Isabel, 47104 Valladolid (ES)

(56) References cited:
- EP-A- 0 615 436
- EP-A- 1 022 014
- EP-A- 1 118 319
- WO-A-00/48556
- WO-A-01/78670
- WO-A-02/34219
- WO-A-97/14396
- WO-A-99/03447
- WO-A-99/13823
- DE-A- 10 056 909
- US-A- 5 601 620
- US-A- 5 942 216
- US-A- 6 315 989
- Fey/Otte:Wörterbuch der Kosmetik, 4th Edition, 1997,pages 119, 120 and 240
- K.-H.Schrader: Grundlagen und Rezepturen der Kosmetika, 2nd Edition, 1989, pages 728 and 729

## Description

This invention relates to a treatment composition which reduces bleeding of acidic dyes from hair coloured permanently or semi-permanently with dyeing agents containing acidic dyestuffs.

Hair colouring is a common practice for ages. Oxidative colouration has been widely used for achieving durable, brilliant hair colour. Direct dyes, mainly of cationic character, have also been found their applications for colouring hair for many years. The colours so achieved are brilliant but often lacking durability. Recently, anionic direct dyes have been found to be very powerful for changing hair colour permanently and to achieve long lasting, brilliant-colours. The colouring agents with anionic dyes are so formulated that the optimum conditions are realised for achieving the highest dyestuff penetration into hair possible. European patent application with laid open number EP 1 022 014 describes such compositions comprising anionic dyestuffs, solvents, as aid to enhance penetration of said dyestuffs, and a buffer solution to adjust the pH of the dyeing agent in the range from 2 to 6. Products are found on the professional hair dressing market applying this technology.

US 5,601,620 as well discloses hair colouring agents with acid dye, an organic solvent and at least one polysiloxane as a conditioner. The dyeing compositions disclosed here are having a pH in the range of 1.5 - 4.5.

In practice, number of difficulties are observed when colouring human hair with acidic dyestuff containing colouring agents. One of them is bleeding (colour wash of from hair) of dyestuff from hair afterwards with hair washes with shampooes and conditoning products, i.e. treatments. This is in such a high extend that people having coloured their hair with acidic dyes complaining as according to their judgement with such high extend of bleeding, hair colour is also changing very rapidly. In other words, durability of the colour is judged inappropriately to be very short.

In practice this problem is presumably being solved by washing hair extensively at the end, of the colouring process so that excess of the dyes adsorbed onto the hair surface is washed out. This is often not economical as high amount of dyes already on the surface of the hair is removed which may lead to change of the achieved hair colour. The optimum should certainly be thatfinding an appropriate washing and conditioning compositions that is taking away less dyes from the hair surface which leads to a better durability. In the above mentioned two patent applications this problem is not addressed at all and, certainly, no alternative options are suggested for solving the problem.
EP 1 118 319 A1 discloses compositions for reducing bleeding of anionic dyes from hair. According to the document, compositions comprising an organic solvent selected from aromatic alcohols, lower alkylene carbonates, N-alkylpyrrolidones and formamides, an organic carboxylic acid or a salt thereof, and a further organic solvent selected from a lower alcohol, a polyhydric alcohol and a lower alkyl ether of a polyhydric alcohol with the pH of the composition within the range of 1 - 6, reduce effectively bleeding of anionic dyes.

The present invention deals only with the conditioning compositions, i.e. treatments, to be used after shampooing hair.

In order to solve the aforementioned problem, it has surprisingly been found out that bleeding is very much related to the composition of treatment used for conditioning and/or repair purposes after colouring hair with direct acidic dyes. Cationic emulsions with relatively high level of organic solvent or solvent mixtures and having pH in the range from 1.5 to 5 is found to be very effective to reduce bleeding of acidic dyes from hair. This type of composition can certainly be at the same time colouring composition when dyes are included into preparations.

Those type of treatment compositions are well known and have been used for many years for hair conditioning purposes i.e. improving combability, gloss, smoothness and softness of hair, as well as repair of damages caused by environmental influences and/or chemical processes. Those emulsions are either cationic or non-ionic with other conditioning agents such as silicones, and having acidic pH as low as pH 3.0 depending on the aimed effect. They even contain often organic solvent at low concentration, i.e. up to 5% by weight.

The conditioning composition of the present invention is in the form of an emulsion and comprises
- at least one fatty alcohol
- at least one emulsifier
- at least one cationic conditioning agent
- at least one organic solvent at a concentration of above 5% by weight
- acidic compound selected from organic and/or inorganic acids or their mixtures at a concentration of 0.1 to 5% by weight, calculated to total composition, and
- optionally salt of organic and/or inorganic acids or their mixtures, and composition has a pH between 1.5 and 5,
which reduces the bleeding of acidic dyes from hair.

The conditioning composition of the present invention contains fatty alcohols with the general formula

R₁-OH

where R₁ is a saturated or unsaturated, branched or non-branched fatty acyl chain with 10 - 24 C atoms. Concentration of fatty alcohols is usually less than 20%, preferably less than 15% and more preferably less than 10% by weight calculated to total composition. Typical example to the most useful fatty alcohols are myristyl alcohol, palmityl alcohol, cetyl alcohol, And the most preferred one is the cetearyl alcohol.

The conditioner composition of the present invention comprises surface-active substances as emulsifiers. These can be anionic and/or nonionic and/or cationic and/or amphoteric or zwitterionic and/or their mixtures incorporated at a concentration ranging between 0.1 - 10 %, preferably 0.1 - 7.5% and more preferably 0.5 - 5% and most preferably 1 - 5% by weight calculated to the total composition. Preferred emulsifiers are of non-ionic and cationic types and the above specified concentrations are given for these emulsifiers. The anionic ones are as a rule not preferred and if their presence is desirable because of any reason, those should form the very minor part, as electrostatic interactions with the cationic material can disturb the stability of those conditioners. Zwitterionic ones are the ones preferred to lesser extent. As the anionics their concentration should be as low as possible.

Suitable nonionic surfactants as emulsifier are compounds from the category of alkyl polyglucosides with the general formula

R₂-O-(CH₂CH₂O)ₙ-Z_{x'}

wherein R₂ is an alkyl group with 8 to 20, preferably 10 to 14 carbon atoms, Z_{X} is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Other additionally useful non-ionic surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Additionally useful surfactants are amineoxides. Such amineoxides are state of the art, for example C₁₂-C₁₈ alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain. Suitable amineoxides are on the market, for example, under the trade names "Ammonyx®, "Aromox®" or "Genaminox®".

Further suitable surfactant components are fatty acid mono- and dialkanolamides, such as coco fatty acid monoethanolamide and myristic fatty acid monoisopropanolamide.

Cationic surfactants (as emulsifiers) and/or hair conditioning agents are also part of the coloring compositions of the present invention which are represented with the general formula below: 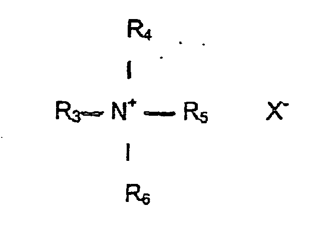 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has typical value of 0 - 4 or

R₈ CO O (CH₂)ₙ

where R₈ is saturated or unsaturated, branched or non-branched alkyl chain with 7-21 C atoms and n has typical value of 0-4 or
R₄ is H or unsaturated or saturated, branched or non-branched alkyl chain with 1 - 22 C atoms or

R₇ CO NH (CH₂)ₙ

or

R₈ CO O (CH₂)ₙ

where R₇, R₈ and n are same as above.

R₅ and R₆ are independent from each other H or lower alkyl chain with 1 to 4 Carbon atoms, and X is chloride, bromide, methosulfate.

Suitable cationic surfactants and or conditioning agents are, for example, long-chain quaternary ammonium compounds which can be used alone or in admixture with one another, such as cetyl trimethyl ammonium chloride, dimethyl diethyl ammonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate.

Amphoteric or zwitterionic surfactants are, not really the preferred ones and may be present at minor quantities, in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 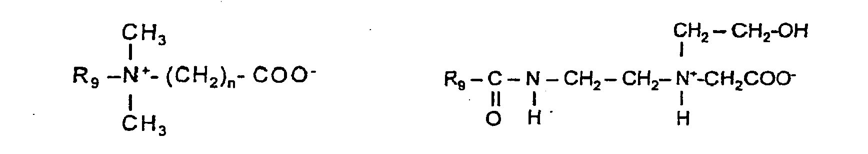 wherein R₉ is a C₈-C₁₈-alkyl group and n is 1 to 3,
sulfobetaines of the structure 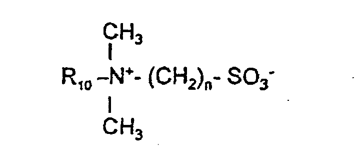 wherein R₁₀ is a C₈-C₁₈-alkyl group and n is 1 to 3,
and amido alkyl betaines of the structure, 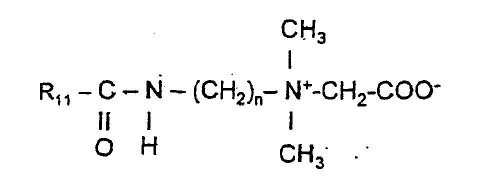 wherein R₁₁ is a C₈-C₁₈-alkyl group and n is 1 to 3.

Preferred are fatty acid amidoalkyl betaines, in particular cocoamidopropyl betaine, and cocoamphoacetate and -propionate, in particular the sodium salts thereof.

Concentration of those amphoteric or zwitterionic surfactants used as emulsifiers should in any case not exceed 1% by weight calculated to the total composition.

Anionic surfactants may, though not preferred, also be part of the composition of the present invention as emulsifiers. Those are of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-and dialkyl phosphates constituting mild, skin-compatible detergents.

Suitable surfactants of the carboxylate type are alkyl polyether carboxylic acids and the salts thereof of the formula

R₁₂-(C₂H₄O)ₙ-O-CH₂COOX,

wherein R₁₂ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 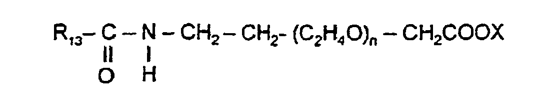 wherein R₁₃ and X have the above meanings, and n is in particular a number from 1 to 10, preferably 2.5 to 5. Such products have been known and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

An overview of the anionic surfactants can furthermore be found in the monography of K. Schrader, "Grundlagen und Rezepturen der Kosmetika", 2^{nd} Ed.(1989, Hüthig Buchverlag), pp. 595-600 and pp. 683 to 691. Those are mainly used in liquid body cleansing compositions, and at the same time especially suitable for conditioning compositions of the present invention as emulsifiers.

Concentration of anionic surfactants should also not exceed 1 % by weight calculated to total composition.

Of special importance here are solvents which are,used as solubilizers and at the same time which enhances penetration of the anionic dyes into the hair, which is necessary in order to achieve intensive long lasting colorations. These "penetration enhancers" are, for example, benzyloxyethanol, benzyl alcohol, phenoxy ethanol, phenoxy isopropanol, methyl phenoxy ethanol, benzyl glycerol, N-benzyl formide, benzyl urea, N-methyl pyrrolidone, N-ethyl pyrrolidone, cinnamyl alcohol, phenethyl-alcohol, p-methyl benzyl alcohol, butyl cellosolve, methyl carbitol, ethyl carbitol, propyl carbitol, butyl carbitol, diethylene glycol, diethyl ether and dipropylene glycol diethyl ether. It is though that because of the penetration enhancing ability, those play an important role in reducing bleeding. Especially preferred ones are ethanol, benzyloxyethanol and benzyl urea.

Concentration of those solvents is at least 5%, preferably at least 7,5% by weight calculated to total composition. In any case the solvent content of the composition of the present invention should not exceed 20%, preferably 15% by weight calculated to total composition.

Treatment composition of the present invention comprises organic and/or inorganic acids or their mixtures. Examples to organic acids are citric acid, lactic acid, tartaric acid, malic acid, maleic acid, benzoic acid, fumaric acid, levulinic acid, butyric acid, valeric acid, oxalic acid, succinic acid, mandelic acid, glycolic acid or inorganic acids such as hydrochloric acid, phosphoric acid, sulphuric acid, nitric acid. Concentration of the organic or inorganic acids and their mixtures should be adjusted in a way that so obtained treatment composition has a pH value between 1.5 and 5, preferably 2 - 4.5 and more preferably 2 - 3.5. Concentration for acids can be preferably 0.1 - 3% by weight and more preferably 0.2 - 2.5% by weight. The pH of the treatment composition can also be adjusted to the required pH by using an alkaline solution such as sodium hydroxide, potassium hydroxide in the case that at the selected acid concentration pH of the composition is lower than that of the aimed value.

Conditioning composition can also contain cationic polymers as a conditioning agents. Those are cationic cellulose type polymers know as Polymer JR type from Amerchol such as Polyquaternium 10 or cationic guar gum known with trade name Jaguar from Rhone-Poulenc and chemically Guar hydroxypropyl trimonium chloride. Furthermore, chitosan and chitin can also be included in the compositions as cationic natural polymers.

Organic or inorganic salts or their mixtures can also be incorporated into conditioning compositions of the present invention. Inorganic ones are typically sodium, potassium, magnesium, calcium, salts of cloride, bromide, phosphate sulfate etc. As organic ones the salts of those acids mentioned above with sodium, potassium, magnesium, calcium, guanidine are the most suitable ones. Concentration of salts is typically in the range of 0.1- 2.5%, preferably in the range of 0.5 - 2%, more preferably in the range of 1 - 2% by weight calculated to total composition.

Treatment composition of this invention can contain other type of known hair conditioning agents. Conditioning agents can be selected from oily substances such as silicone oils either volatile or non-volatile, natural and synthetic oils. Among silicone oils those can be added to the pre-treatment composition include dimethicone, dimethiconol, polydimethylsiloxane, DC fluid ranges from Dow Corning, natural oils such as olive oil, almond oil, avocado oil, sunflower oil, peach oil, jojoba oil, ricinus oil and the synthetic oils, such as mineral oil.

Non-ionic conditioning agents can also be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula I or II

R₁₄ CO (O CH₂ CH₂)ₙ OH

R₁₄ CO (O CH₂ CH₂)ₙ O OC R₁₅

where R₁₄ and R₁₅ are independent from each other saturated, unsaturated or branched or non-branched alkyl chain with 7 to 21 C atoms and n is typically 2-100. Typical concentration range for those conditioners can be 0.05 - 5% by weight, preferably 0.05 - 3.5% by weight and more preferably 0.05 - 2,5% by weight.

Viscosity of compositions is between 1000 mPa.s to 75000 mPa.s measured at 20°C with Brookfield viscosimeter with, for example, Spindle 5 at 5 rpm.

Following examples are to illustrate the invention.

Colouring agent: Elumen RR @ all (commercial product under the brand name Goldwell) is used throughout the tests for showing the effectiveness of the present invention.

### Example 1

| | |
|---|---|
| Ethanol | 15.0 % by wt. |
| Benzyl alcohol | 5.0 |
| Cetrimonium chloride | 0.8 |
| Cetearyl alcohol | 12.0 |
| Silicium oil | 1.5 |
| Sodium hydroxide (32%) | 0.25 |
| Phosphoric acid | q.s. pH 2.0 |
| Water | ad 100.0 |

The conditioner is prepared by melting/dissolving first cetearyl alcohol, cetrimonium chloride and silicone oil in part of the water at 80°C. Theother ingredients are dissolved in 80°C water. Afterwards both portions are combined and emulsified with the aid of homogenizer at for example 2000 rpm. The composition is cooled down and pH is adjusted. Viscosity of the emulsion at 20°C was 35,000 mPa.s measured with a Brookfield viscosimeter, spindle 4 at 5 rpm.

Swatches (2) of hair weighing approximately 1g were dyed for 20 min. at 50°C with the above mentioned coloring agent, then rinsed off and shampooed twice with a commercially available shampoo for coloured hair as well available from the same brand. Thereafter, an emulsion according to Example 1 is applied for 5 min. only onto one of the swatches of the same colour, the other remained untreated. The product is rinsed off and after drying the hair swatches are immersed in 100ml of a 5% sodium laureth sulfate solution (70%, trade name Emal 270) for 15 min. at 45°C. The solutions so obtained are contained very high concentration of dyes and therefore diluted before measuring the extinctions appropriately to bring the extinction valued in the range of a successful measurement, i.e. lower than relative extinction value of 1.0. The extinction of the solutions are subsequently measured at 488 nm after making appropriate corrections for measurements as usual with a suitable spectrophotometer as indicator of the bleeding. From the extinction values, the amount of dye washed out is approximated using a calibration curve.

| | with Example 1 | without Example 1 |
|---|---|---|
| Released amount of Acid Red 52/g of hair | 0,015 | 0.020 |

The similar results are also obtained with a brown red shade of the same brand.

Other examples are as follows showing as well the same effect.

### Example 2:

| | |
|---|---|
| Ethanol | 15.0 % by wt. |
| Benzyl alcohol | 5.0 |
| Cetrimonium chloride | 0.8 |
| Cetearyl alcohol | 10.0 |
| Silicium oil | 1.5 |
| Phosphoric acid | ad pH 2.5 |
| Water | ad 100.0 |

### Example 3:

| | |
|---|---|
| Ethanol | 15.0 % by wt. |
| Benzyl urea | 2.0 |
| Sodium isothionate | 5.0 |
| Cetrimonium chloride | 0.8 |
| Cetearyl alcohol | 10.0 |
| Silicium oil | 1.5 |
| Phosphoric acid | ad pH 3.0 |
| Water | ad 100.0 |

### Example 4:

| | |
|---|---|
| Ethanol | 15.0 % by wt. |
| Benzyloxyethanol | 10.00 |
| Sodium chloride | 5.0 |
| Cetrimonium chloride | 0.8 |
| Behentrimonium chloride | 0.5 |
| Cetearyl alcohol | 12.0 |
| Silicium oil | 1.5 |
| Phosphoric acid | ad pH 2.0 |
| Water | ad 100.0 |

### Example 5:

| | |
|---|---|
| Ethanol | 15.0 % by wt. |
| Benzyl alcohol | 5.0 |
| Guanidinium chloride | 5.0 |
| Cetrimonium chloride | 0.8 |
| Cetearyl alcohol | 12.0 |
| Silicium oil | 1.5 |
| Phosphoric acid | ad pH 2.5 |
| Water | ad 100.0 |

## Claims

1. Conditioning composition in the form of an aqueous emulsion for reducing bleeding of anionic dyes from coloured hair **characterised in that** it comprises
- at least one fatty alcohol, and
- at least one emulsifier, and
- at least one cationic conditioning agent, and
- at least one organic solvent at a concentration of above 5% by weight, calculated to total composition, and acidic compound selected from organic or inorganic acids or their mixtures, at a concentration of 0,1 to 5% by weight, calculated to total composition, and composition has a pH between 1.5 and 5.

2. Conditioning composition according to claim 1 **characterized in that** it comprises emulsifier selected from non-ionic and cationic emulsifiers or their mixtures at a concentration of 0.1 - 10% by weight calculated to total composition.

3. Conditioning composition according to any of the preceding claims **characterized in that** it comprises cationic conditioning agents selected from 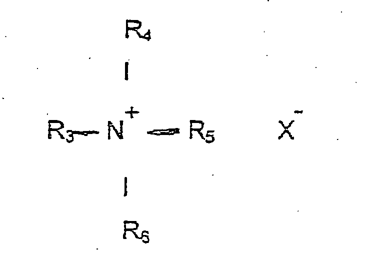 where R₃ is a saturated or unsaturated, branched or non-branched alkyl chain with 8-22 C atoms, or
R₇ CO NH (CH₂)ₙ
where R₇ is saturated or unsaturated, branched or non-branched alkyl chain with 7-21 C atoms and n has value of 0 - 4, or
R₇ CO O (CH₂)ₙ
where R₇ is saturated or unsaturated, branched or non-branched alkyl chain with 7-21 C atoms and n has value of 0 - 4
and
R₄ is H or unsaturated or saturated, branched or non-branched alkyl chain with 1 - 22 C atoms or
R₇ CO NH (CH₂)ₙ
where R₇ is saturated or unsaturated, branched or non-branched alkyl chain with 7-21 C atoms and n has value of 0- 4, or '
R₈ CO O (CH₂)ₙ where R₈ is saturated or unsaturated, branched or non-branched alkyl chain with 7-21 C atoms and n has value of 0 - 4
and
R₅ and R₆ are independent from each other H or lower alkyl chain with 1 to 4 Carbon atoms, and X is chloride, bromide, methosulfate.

4. Conditioning composition according to any of the preceding claims **characterized in that** it comprises one or more fatty alcohol at a concentration of less than 20% by weight calculated to total composition

5. Conditioning composition according to any of the preceding claims **characterized in that** it additionally comprises salts of organic or inorganic acids or their mixtures at a concentration of 0.1- 2.5% by weight calculated to total composition.

6. Process for conditioning hair coloured with acidic dyes **characterised in that** after shampooing hair appropriate amount of conditioner according to any of the preceding claims is applied onto hair and after a short processing time rinsed off again for reducing bleeding of acidic dyes from hair.

## Patentansprüche

1. Haarpflegezusammensetzung in Form einer wässrigen Emulsion zur Reduzierung des Ausblutens anionischer Farben aus dem gefärbten Haar, insbesondere **dadurch gekennzeichnet, dass** sie
- mindestens einen Fettalkohol, und
- mindestens einen Emulgator, und
- mindestens einen kationischen Pflegewirkstoff, und
- mindestens ein organisches Lösungsmittel in einer Konzentration von mehr als 5 Gew.-%, berechnet auf die Gesamzusammensetzung, und
- eine saure Verbindung, ausgewählt aus organischen oder anorganischen Säuren oder ihren Mischungen in einer Konzentration von 0,1 - 5 Gew.-%, berechnet auf die Gesamtzusammensetzung, enthält.

2. Haarpflegezusammensetzung nach Anspruch 1, **dadurch gekennzeichnet, dass** sie einen aus nichtionischen oder kationischen Emulgatoren oder deren Mischungen ausgewählten Emulgator in einer Konzentration von 0,1 - 10 Gew.-%, berechnet auf die Gesamtzusammensetzung, enthält.

3. Haarpflegezusammensetzung nach Ansprüchen 1 bis 3, **dadurch gekennzeichnet, dass** sie kationische Pflegewirkstoffe enthält, die ausgewählt sind aus: 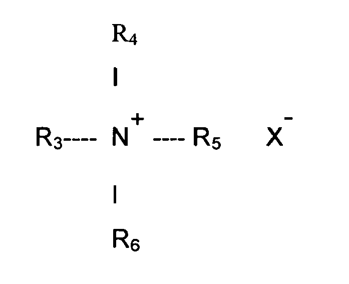 wobei R₃ eine gesättigte oder ungesättigte, verzweigte oder nicht verzweigte Alkyl-Kette mit 8 - 22 C-Atomen ist, oder
R₇ CO NH (CH₂)ₙ
wobei R₇ eine gesättigte oder ungesättigte, verzweigte oder nicht verzweigte Alkyl-Kette mit 7 - 21 C-Atomen ist und ₙ einen Wert von 0 - 4 hat, oder
R₇ CO O (CH₂)ₙ
wobei R₇ eine gesättigte oder ungesättigte, verzweigte oder nicht verzweigte Alkyl-Kette mit 7 - 21 C-Atomen ist und ₙ einen Wert von 0 - 4 hat
und
R₄ gleich H ist oder aus einer ungesättigten oder gesättigten, verzweigten oder nicht verzweigten Alkyl-Kette mit 1 - 22 C-Atomen besteht, oder
R₇ CO NH (CH₂)ₙ
wobei R₇ eine gesättigte oder nicht gesättigte, verzweigte oder nicht verzweigte Alkyl-Kette mit 7 - 21 C-Atomen ist und ₙ einen Wert von 0 - 4 hat, oder
R₈ CO O (CH₂)ₙ
wobei R₈ eine gesättigte oder ungesättigte, verzweigte oder nicht verzweigte Alkyl-Kette mit 7 - 21 C-Atomen ist und ₙ einen Wert von 0 - 4 hat,
und
R₅ und R₆, unabhängig voneinander, H oder eine kurze Alkyl-Kette mit 1 bis 4 Kohlenstoff-Atomen sind, und X Chlorid, Bromid und/oder Methosulfat ist.

4. Haarpflegezusammensetzung nach Ansprüchen 1 bis 4, **dadurch gekennzeichnet, dass** sie einen oder mehrere Fettalkohole in einer Konzentration von weniger als 20 Gew.-%, berechnet auf die Gesamtzusammensetzung, enthält.

5. Haarpflegezusammensetzung nach Ansprüchen 1 bis 5, **dadurch gekennzeichnet, dass** sie zusätzlich Salze von organischen oder anorganischen Säuren oder deren Mischungen in einer Konzentration von 0,1 - 2,5 Gew.-%, berechnet auf die Gesamtzusammensetzung, enthält.

6. Verfahren zum Behandeln von mit sauren Farbstoffen gefärbten Haaren, **dadurch gekennzeichnet, dass** nach dem Shampoonieren eine angemessene Menge der Haarpflegezusammensetzung nach einem der Ansprüche 1 bis 6 auf das Haar aufgetragen und nach einer kurzen Einwirkzeit wieder abgespült wird, um das Ausbluten saurer Farbstoffe aus dem Haar zu reduzieren.

## Revendications

1. Composition de conditionnement qui présente la forme d'une émulsion aqueuse destinée à réduire l'exsudation de colorants anioniques par des cheveux colorés, **caractérisée en qu'**elle comprend :
- au moins un alcool gras,
- au moins un émulsifiant,
- au moins un agent cationique de conditionnement,
- au moins un solvant organique à une concentration supérieure à 5 % en poids par rapport à la composition totale, et
- un composé acide sélectionné parmi les acides organiques ou minéraux ou leurs mélanges à une concentration de 0,1 à 5 % en poids par rapport à la composition totale.

2. Composition de conditionnement selon la revendication 1, **caractérisée en ce qu'**elle comprend un émulsifiant sélectionné parmi des émulsifiants non ioniques et cationiques ou leurs mélanges à une concentration de 0,1 à 10% en poids par rapport à la composition totale.

3. Composition de conditionnement selon l'une quelconque des revendications précédentes, **caractérisée en ce qu'**elle comprend des agents cationique de conditionnement sélectionnés parmi : 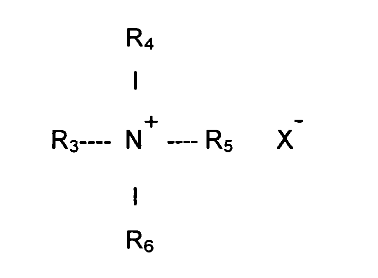 dans laquelle R₃ est une chaîne alkyle saturée ou insaturée, ramifiée ou non ramifiée, qui compte de 8 à 22 atomes de C, ou
R₇ CO NH (CH₂)ₙ
dans laquelle R₇ est une chaîne alkyle saturée ou insaturée, ramifiée ou non ramifiée qui compte de 7 à 21 atomes de C, n ayant une valeur comprise entre 0 et 4, ou
R₇ CO O (CH₂)ₙ
dans laquelle R₇ est une chaîne alkyle saturée ou insaturée, ramifiée ou non ramifiée qui compte de 7 à 21 atomes de C, n ayant une valeur comprise entre
0 et 4, et
R₄ représente H ou une chaîne alkyle insaturée ou saturée, ramifiée ou non ramifiée, qui compte de 1 à 22 atomes de C, ou
R₇ CO NH (CH₂)ₙ
dans laquelle R₇ est une cha1ine alkyle saturée ou insaturée, ramifiée ou non ramifiée qui compte de 7 à 21 atomes de C, n ayant une valeur comprise entre 0 et 4, ou
R₈ CO O (CH₂)ₙ
dans laquelle R8 est une chaîne alkyle saturée ou insaturée, ramifiée ou non ramifiée qui compte de 7 à 21 atomes de C, n ayant une valeur compris entre 0 et 4, et
R₅ et R₆ représentent indépendamment l'un de l'autre H ou une chaîne alkyle inférieure qui compte de 1 à 4 atomes de carbone, et X représentant un chlorure, bromure ou méthosulfate.

4. Composition de conditionnement selon l'une quelconque des revendications précédentes, **caractérisée en ce qu'**elle comprend un ou plusieurs alcools gras à une concentration inférieure à 20 % en poids par rapport à la composition totale.

5. Composition de conditionnement selon l'une quelconque des revendications précédentes, **caractérisée en ce qu'**elle comprend en supplément des sels d'acides organiques ou minéraux ou leurs mélanges à une concentration de 0,1 à 2,5 % en poids par rapport à la composition totale.

6. Procédé de conditionnement des cheveux colorés à l'aide de colorants acides, **caractérisé en ce qu'**après le shampooing des cheveux, une quantité appropriée d'agent de conditionnement selon l'une quelconque des revendications précédentes est appliquée sur les cheveux et est rincée après un court temps de traitement pour réduire l'exsudation des colorantes acides par les cheveux.
